(19) Europäisches Patentamt

European Patent Office

Office européen des brevets

(11) Veröffentlichungsnummer: **0 227 050 B1**

## EUROPÄISCHE PATENTSCHRIFT

(45) Veröffentlichungstag der Patentschrift: **10.04.91**

(51) Int. Cl.5: **A61K 9/20**, A61J 3/06

(21) Anmeldenummer: **86117669.1**

(22) Anmeldetag: **18.12.86**

(54) **Verfahren zur Herstellung von einzeln dosierten Darreichungsformen sowie die danach erhältlichen Darreichungsformen.**

(30) Priorität: **19.12.85 DE 3545090**

(43) Veröffentlichungstag der Anmeldung:
**01.07.87 Patentblatt 87/27**

(45) Bekanntmachung des Hinweises auf die
Patenterteilung:
**10.04.91 Patentblatt 91/15**

(84) Benannte Vertragsstaaten:
**ES GR**

(56) Entgegenhaltungen:
**CH-A- 284 791**
**FR-A- 2 179 044**
**FR-A- 2 343 473**
**FR-A- 2 530 421**

(73) Patentinhaber: **Capsoid Pharma GmbH**
**Im Brühl 4**
**W-7128 Lauffen a.N.(DE)**

(72) Erfinder: **Pins, Heinrich, Dr.**

**deceded(DE)**
Erfinder: **Schmitz, Christiane**
**Rieslingstrasse 38**
**W-7128 Lauffen a.N.(DE)**

(74) Vertreter: **Hagemann, Heinrich, Dr.**
**Dipl.-Chem. et al**
**Patentanwälte HAGEMANN & KEHL Ismaninger Strasse 108 Postfach 860329**
**W-8000 München 86(DE)**

## Beschreibung

Die Erfindung betrifft ein Verfahren zur Herstellung einzeln dosierter Darreichungsformen, wobei eine bei erhöhter Temperatur plastische, Wirkstoffe und übliche Additive enthaltende, auf einer Sol-/Gel-Beziehung beruhende Rezepturmasse eines oberhalb der Körpertemperatur liegenden Erweichungsbereiches auf über Körpertemperatur erwärmt und mit einer Viskosität von mehr als 3000 mPa.s unter Druckeinwirkung auf eine ruhende oder bewegte Unterlage gebracht oder in aus Folien hergestellte Blister oder Hohlformen eingebracht und die Rezepturmasse hierdurch und unter Abkühlen in eine Darreichungsform überführt wird.

Als herkömmliche, weitverbreitete, feste Darreichungsformen für die Applikation von Arzneimitteln, Diätetika und dergleichen sind im wesentlichen Tabletten, Dragees, Weich- und Hartgelatinekapseln bekannt. Diese genannten Darreichungsformen bedürfen alle in einem ersten Schritt der Formgebung und in einem zweiten Schritt einer produktgemäßen Verpackung, die sowohl den Anforderungen des Produktes (z.B. Stabilität) als auch den Konsumentenwünschen (z. B. Attraktivität) gerecht wird.

Diese schrittweise Herstellung eines Produktes bis zur Endverpackung muß als kostspieliger Nachteil empfunden werden, wenn es gelingt, zu brauchbaren Produkten zu kommen, die nicht mehr der gesonderten Formgebung bedürfen, sondern durch ihre Verpackung letztlich ihre Form erhalten oder gar aufgrund ihrer Konsistenz durch einfaches, dosierendes Aufbringen auf eine Unterlage zu der gewünschten Form "verlaufen" und sich verfestigen. Insgesamt müssen zweistufige Verfahrensweisen als nachteilig empfunden werden, wenn einfache Wege möglich sind.

Man könnte nun davon ausgehen, daß Verfahren zum Füllen von Formen altbekannte und verbreitete Prozeduren sind, denen kein Neuheitswert mehr anhaftet. In der Tat finden sich in der Patentliteratur Hinweise zu diesem Thema, auch für den pharmazeutischen Bereich, wie nachfolgend gezeigt.

Die DE-B- 1 017 322 stellt ein Verfahren in den Vordergrund, bei dem eine kombinierte Gießform und Verpackung zum Gießen und Versand von verkaufs- und gebrauchsfertigen Formlingen aus Medikamenten und ähnlichen Stoffen, wie Suppositorien, Pastillen oder dergleichen, herangezogen werden. Jedoch werden jeweils zwei oder mehr gelenkig miteinander verbundene Formteile benötigt, die jedes für sich mehr als die halbe Dicke des späteren Gießteiles aufweisen müssen, um im Sinne des bekannten Vorschlags wirksam werden zu können. Die Abtrennung einer verpackten Einzeldosis ist nicht möglich.

Der Schiebedeckel muß in seiner gesamten Länge von der Packung entfernt werden, um das geforderte Auseinanderklappen der Formteile zur Entnahme der Formlinge zu ermöglichen. Dadurch tritt zwangsläufig eine Kontamination der in der Packung verbleibenden Stücke durch Schmutz und Mikroorganismen ein. Die DE-B- 1 017 322 erwähnt ebenfalls das Befüllen der Formteile unter Zuhilfenahme eines Gießrahmens, der ein randvolles Füllen der Formen ermöglichen soll. Nach dem Gießen überstehendes und danach erstarrtes Material kann abgeschabt und wiederverwendet werden. Dieser Prozeß des Füllens kann allein aufgrund seiner Dosierungsungenauigkeit kein pharmazeutisch relevantes Verfahren sein, aus dem Medikamente resultieren, die den heutigen Ansprüchen gerecht werden.

Entsprechendes gilt auch für den sich aus der DE-C- 1 947 684 ergebenden Vorschlag zur Herstellung von verpackten Darreichungsformen. Dieser Vorschlag stellt eine Weiterentwicklung des Standes der Technik nach der DE-B- 1 017 322 insofern dar, als statt des erwähnten Gießrahmens eine durchlöcherte Folie ein sauberes Befüllen der einzelnen Formen ermöglichen soll. Auch hier handelt es sich um ein komplexes, diskontinuierliches und hygienisch bedenkliches Vorgehen, das somit nicht zufriedenstellen kann.

Die Technik des Gießens fetthaltiger Körper ist in der Zwischenzeit weiterentwickelt worden. So werden seit einiger Zeit aus Folien vorgeformte, vereinzelbare Hohlformen mit geschmolzener Suppositorienmasse gefüllt. Nach Passieren eines Kühltunnels wird die bis dahin offene Form versiegelt. Mit kostspieligen, automatisierten Maschinen werden Stundenleistungen von etwa 25 000 Suppositorien erreicht. Nachteilig bei diesem Prozeß, der sich auf Fettmassen mit Schmelzpunkten unter 40 °C beschränkt, ist die Gefahr der Sedimentation fester Stoffe beim Gieß und Abkühlvorgang infolge der niedrigen Viskosität der geschmolzenen Fettmasse. Eine weitere Gefahr stellt die Veränderung der physikalischen Parameter der eingesetzten Fette durch Aufschmelzen und Wiederabkühlen dar. Es kann zu Schmelzpunktveränderungen und auch zu Brüchigkeit der gegossenen Körper kommen.

Es hat Ansätze gegeben, die Kenntnis des Gießens von Suppositorien auch auf, oral applizierbare Dosisformen zu übertragen. So beschreibt die DE-A- 27 10 307 (entspricht FR-A- 2 343 473) ein Verfahren zur Herstellung und Verpackung von festen pharmazeutischen Dosiseinheiten, die in Tablettenform hergestellt werden, was sich dadurch erreichen läßt, daß das Gemisch auf ein fortlaufendes Band aus unterschiedlichem Material, das tablettenförmige Vertiefungen besitzt, aufgebracht wird. Als Trägersubstanz werden Fette. Fettgemische, andere lipoide Substanzen und lipoide Komponenten erwähnt, deren Schmelz-

EP 0 227 050 B1

punkt über 37°C, vorzugsweise über 43°C liegen soll. Die für die vorliegende Erfindung eigentlich wichtige Aussage der DE-A-27 10 307 liegt in dem Satz, daß das geschmolzene Gemisch aus Trägersubstanz und aktiver Komponente mit oder ohne Meßvorrichtung in die Vertiefungen "gegossen" wird. Dies kann nur bedeuten, daß es sich um ein niedrigviskoses, verflüssigtes Substanzgemisch handelt, daß sich noch gießen läßt, das dadurch aber die Gefahr der Sedimentation von Feststoffen in sich birgt und sich selbst in der Verwendung der Trägerstoffe eingrenzt.

Das Verfahren der DE-A-27 10 307 kommt in seiner Beschreibung nicht über die Verwendung von relativ niedrig schmelzenden Fetten und Wachsen hinaus, die als Matrix für den oder die Wirkstoffe dienen. Es bleibt offen, wie diese Körper ohne Bruch aus den Vertiefungen der Folien zu entfernen sind, wie sie als Fettkörper schmecken und welche Freisetzungsraten zu erwarten sind. Zumindest ist bis heute kein Präparat bekannt geworden, das Marktgeltung erlangt hätte.

Die FR-A-2 179 044 beschreibt ein Verfahren, mit dem man zu ähnlichen Körpern gelangen kann, wie sie eingangs beschrieben wurden. Diesem Verfahren haften aber verschiedene gravierende Nachteile an. Dabei ist es wesentlich, daß es das Verfahren nach der FR-A- 2 179 044 nicht gestattet, während seiner Durchführung die Dosiermenge mit exakter Dosiergenauigkeit steuerbar zu korrigieren. Auch benötigt es einen separaten Schritt der Formgebung. Die geformte Dosiereinheit muß aus dem Formwerkzeug entfernt und in eine Verpackung überführt werden. Ein entscheidendes Merkmal der nach dem Verfahren der FR-A- 2 179 044 zu fertigenden Körper ist die zwingende Notwendigkeit des Wasserzusatzes. Es wird als wesentlich angesehen, daß im fertigen Erzeugnis eine Restfeuchtigkeit von 5 bis 20 % verbleibt.

Die Kenntnis der bisher geschilderten Patentliteratur und verwandter Veröffentlichungen kann nicht darüber hinwegtäuschen, daß die vorgestellten Ergebnisse nicht befriedigen. Zum einen ist die Verwendung von Trägerstoffen auf solche Materialien limitiert, die als Fette oder Wachse einen relativ niedrigen Schmelzpunkt haben, zum anderen ist das Einbringen der Masse in die Formen auf Gießvorgänge beschränkt.

Der Erfindung lag daher die Aufgabe zugrunde, ein Verfahren vorzuschlagen, daß es gestattet, in einfacher und wirtschaftlicher Weise qualitativ hochwertige, einzeln dosierte Darreichungsformen herzustellen, wobei der separate Schritt der Formgebung entfällt und während des Verfahrens die Dosiermenge mit exakter Dosiergenauigkeit gestaltet werden und das Erfordernis des Wasserzusatzes entfallen kann.

Erfindungsgemäß wird diese Aufgabe dadurch gelöst, daß die zu dosierende warme Rezepturmasse aus einem Aufheiztank in ein mit hohem Druck arbeitendes heizbares Dosiergerät geführt wird, das ansteuerbare Ventile besitzt, gegen die Kolben- oder Zahnradpumpen einen gleichmäßig hohen Druck aufbauen, und daß durch zeitlich genau begrenztes Öffnen der Ventile eine gleichbleibende Menge der zu dosierenden warmen Rezepturmasse auf die Unterlage oder in die Blister oder Hohlformen ausgetragen wird.

Wesentliches Kennzeichen der Erfindung ist es demzufolge, daß die zu dosierende Rezepturmasse einen Erweichungsbereich aufweist, der oberhalb der Körpertemperatur liegt. Somit hat die erfindungsgemäß dosierte Rezepturmasse den Charakter eines Thermoplasts bzw. eines Plastomers. Es handelt sich daher um Materialien, die bei gewöhnlicher Temperatur im wesentlichen nicht dauerhaft verformbar sind. Es kann sich daher um bei gewöhnlicher Temperatur harte oder sogar spröde Materialien handeln, die dann bei Wärmezufuhr reversibel erweichen und mechanisch verhältnismäßig leicht verformbar werden, um schließlich bei höheren Temperaturen in den Zustand der viskosen Masse überzugehen. In jedem Fall muß dabei die Forderung der Erfindung erfüllt sein, daß die Rezepturmasse einen oberhalb der Körpertemperatur liegenden Erweichungsbereich aufweist. Es versteht sich für den Fachmann von selbst, daß diese Forderung bzw. die Charakteristik des Thermoplasts im wesentlichen durch die nachfolgend noch detaillierter beschriebene Trägermasse bestimmt wird. Einfluß hierauf haben allerdings auch Additive, wie plastifizierende Additive etc. Die weitere Forderung der Erfindung besteht dann darin, daß die Mindestviskosität der Trägermasse 3.000 mPa.s beträgt. Dieser Viskositätswert kann weit überschritten werden, so bei mehr als 50.000 mPa.s oder sogar mehr als 100.000 mPa.s liegen und bis 500.000 mPa.s betragen.

Mit besonderem Erfolg läßt sich das erfindungsgemäße Verfahren mit Dosiergeräten durchführen, die Viskositäten von 500.000 mPa.s zu bewältigen imstande sind, da einfache Pump- und Gießvorgänge nicht mehr in Frage kommen.

Als Dosiergeräte können insbesondere heizbare, mit hohem Druck arbeitende Aggregate verwendet werden, die ansteuerbare Ventile besitzen, gegen die Kolben- oder Zahnradpumpen einen gleichmäßig hohen Druck aufbauen. Durch zeitlich genau begrenztes Öffnen der Ventile wird eine stets gleichbleibende Menge der abzufüllenden Rezepturmasse ausgetragen. Eingebaute Düsen oder Füllnadeln sorgen für eine saubere Füllung der Hohlformen und Blister oder ein exaktes Auftragen auf Unterlagen. Der Druck, unter dem die Dosierung erfolgt, liegt in der Regel über 2 bar und hängt von der Viskosität der zu dosierenden erwärmten Rezepturmasse ab. Vorteilhafterweise liegt er zwischen 10 und 70 bar, insbesondere zwischen

3

35 und 55 bar.

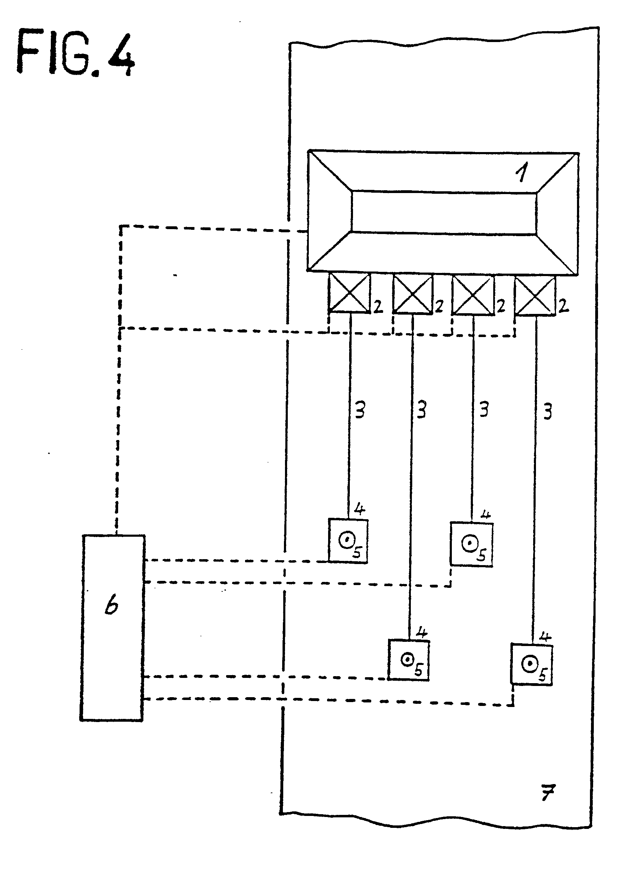

Grundgeräte des hier beschriebenen Typs von Abfüllanlagen werden z. B. von den Firmen Planatol-Werk oder Gerhards-Dosiertechnik angeboten und durch die Figur 4 erläutert. Wesentliche Elemente solcher Geräte sind ein heizbarer Vorwärme- und Aufheiztank 1, aus dem die warme, zu dosierende Masse mit Hilfe von in den Tank ragenden Zahnradpumpen 2 unter regulierbarem Druck und Bypass-Steuerung in heizbare Hochdruckschläuche 3 gebracht wird. Die Geräte können einen oder mehrere Ausgänge für Schlauchanschlüsse haben. In den Schläuchen kann ein Druck bis zu 160 bar anstehen. Die Schläuche münden in heizbaren Dosierköpfen 4, die mit federgelagerten Kugelventilen und Düsen 5 ausgerüstet sind. Mit Hilfe gesteuerter Preßluft werden nach Millisekunden gerechnete Ventilöffnungs- und -schließspiele bewirkt, wobei je nach anstehendem Druck vor dem Ventil bei gleichen Drucken und Öffnungszeiten gleichviel Masse ausgetragen wird. Eventueller Fadenbildung der zu verspritzenden Massen kann durch unterschiedliche Düsengrößen und Temperaturveränderung an den Dosierköpfen begegnet werden. Neben als Injektionsnadeln ausgebildeten Düsen können auch Kegeldüsen, Zwei- und Mehrfachdüsen sowie Flächendüsen eingesetzt werden. Eine Regel- und Steuerungselektronik 6 sorgt für die Überwachung und Einhaltung vorgegebener Werte für Druck und Temperatur. Durch Synchronisationsschaltung können Drehzahl der Zahnradpumpen und Geschwindigkeit eines die Formen tragenden Bandes angeglichen werden.

Um die speziellen Zwecke der vorliegenden Erfindung ganz besonders zu erfüllen, werden an den Grundgeräten Modifikationen vorgenommen, die es z. B. erlauben, die Spritzköpfe so anzuordnen, daß sie an den vorbeigleitenden Formen Mehrfachspritzungen pro Form vornehmen können. Gleichzeitig werden sie verfahrbar installiert, um Füllgutproben zu analytischen Zwecken, z. B. zur Bestimmung der Homogenität des Füllgutes oder des Spritzgewichtes, entnehmen zu können.

Da mehrere Spritzköpfe gleichzeitig eingesetzt und von einer Zentraleinheit gesteuert werden können, lassen sich ohne Mühe 100.000 und mehr Formen pro Stunde füllen. Eine Limitierung des Ausstoßes wird lediglich durch den mechanischen Vorschub der Formen gegeben; jede einzelne Spritzdüse könnte mehr als 1000 Schaltspiele pro Minute ausführen. So wird mit dieser Art von Abfüllsystem eine vielfach höhere Leistung als mit den bisher für das Füllen vorgeformter Formen gebräuchlichen Geräten erreicht.

Die Dosiergenauigkeit der einzelnen Spritzköpfe kann durch separates Ausfahren aus der Füll-Linie und durch separates Spritzen in ein Wägeschälchen überprüft werden. Bei einem Ausstoß von 70.000 gefüllten Tiefziehformen pro Stunde wurden bei einem Füllgewicht von 500 mg eine Dosiergenauigkeit$< \pm$ 2 % und eine relative Standardabweichung$<$ 1 % gefunden. Für die laufende Kontrolle während des Abfüllprozesses eignen sich Laetus-Geräte, die jede Form auf genaue Füllung überwachen. Darüber hinaus können die fertig versiegelten Streifen des weiteren noch über eine ± Waage geleitet werden.

Anhand des oben beschriebenen erfindungsgemäßen Vorgehens lassen sich, was wesentlich ist, hochkonzentrierte Lösungen oder Suspensionen von Wirkstoffen mit wenig Trägerstoff verarbeiten, die sich dennoch mit großer Genauigkeit abfüllen lassen. Im Falle von hochviskosen Lösungen oder Suspensionen tritt dabei die bereits angesprochene Sedimentationsneigung weitgehend zurück. Die Abfüllgeräte erlauben, Massen mit Viskositäten auch oberhalb 500.000 mPa.s zu verarbeiten. So kommt man zu kleinen platzsparenden,leicht zu schluckenden Einzeldosisformen.

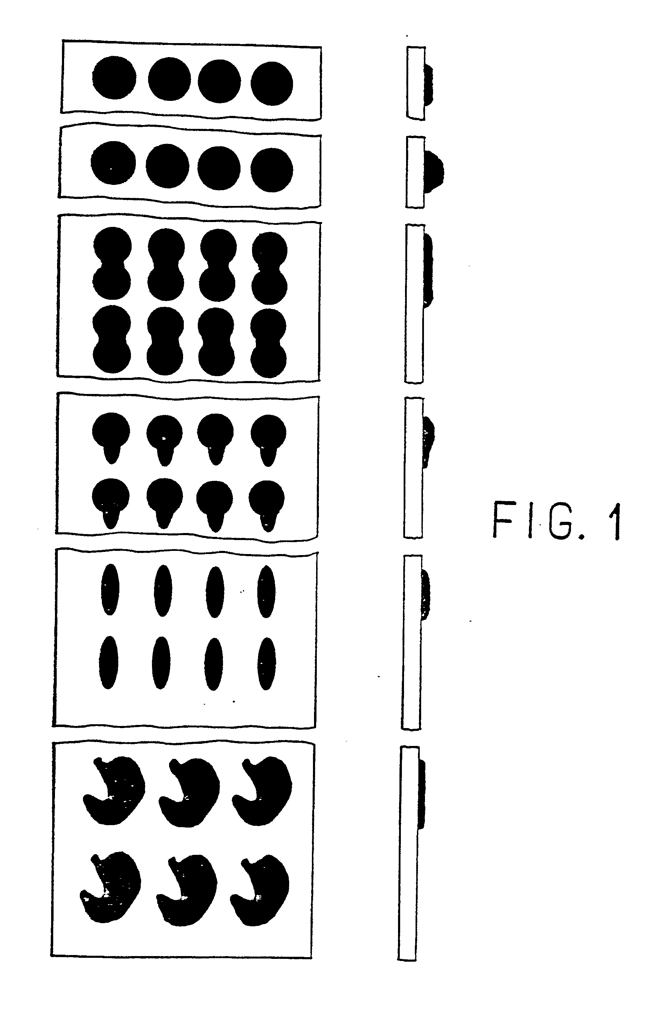

Mit Hilfe der soeben beschriebenen Dosiertechnik gelingt es nun, Dosisformen herzustellen, die im einfachsten Falle ohne jedes Formwerkzeug auf eine Unterlage - entweder gekühlt, bei Raumtemperatur oder darüber hinaus erwärmt, entweder in Ruhe oder bewegt - gespritzt werden. Viskosität und Temperatur der zu verarbeitenden Massen bestimmen dann die Form des erwünschten Körpers. Auf diese Weise erhält man entweder flach verlaufende "Plätzchen" von tablettenähnlichem Aussehen oder mehr hütchenförmige Produkte. Wird die Unterlage, etwa ein Transportband, während des Spritzvorganges bewegt, so gelangt man zu Stäbchen oder Streifen. Bei gleichzeitiger Bewegung des Sprühkopfes in unterschiedliche Richtungen, z. B. mit Hilfe einer Excenterscheibe, erhält man die verschiedenartigsten Produktformen, die z. B. die Gestalt von inneren Organen, wie Herz oder Magen, annehmen können und auf diese Weise auf das Indikationsgebiet des erzeugten Produktes hinweisen. (Fig. 1).

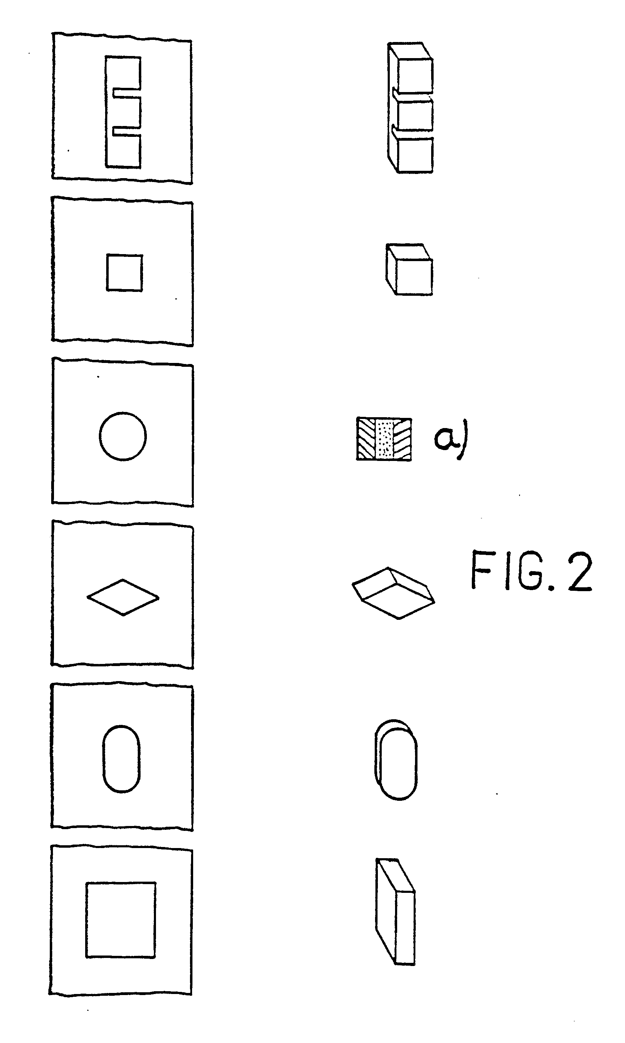

In logischer Verfolgung des erfindungsgemäßen Gedankens geht eine weitere Ausführungsform des Verfahrens so vor, daß sogenannte Durchdrückpackungen gefüllt werden. Danach werden aus Folien unterschiedlichster Art Blister oder "Näpfe" gezogen, die befüllt und mit einer Deckfolie, die meist aus beschichtetem Aluminium besteht, abgesiegelt werden. Die eingeschlossene Rezepturmasse wird durch die Deckfolie gedrückt und ist somit verfügbar. Auch nach dieser Ausgestaltung des erfindungsgemäßen Verfahrens lassen sich viele nach Form und Größe unterschiedliche Darreichungsformen herstellen (Fig. 2). Es ist zweckmäßig, den Näpfen eine leicht konische Form zu geben, um das Herausdrücken des Produktes zu erleichtern.

Die eleganteste Anwendung des erfindungsgemäßen Verfahrens ist die Befüllung von Hohlformen, also bis auf einen Einfülltrichter rundum geschlossenen Formen jeder Größe und Ausformung.

Zur Herstellung dieser Hohlformen können im wesentlichen alle handelsüblichen, verform- und versiegelbaren Folien herangezogen werden, soweit sie mit dem einzubringenden Inhalt verträglich sind. Insbesondere eignen sich dazu thermoplastische Kunststoffe, wie vorzugsweise polyethylenbeschichtete PVC-, PP- oder PVDC-Folien, aber auch kunststoffbeschichtete Aluminiumfolien, soweit sie sich zur gewünschten Hohlform verformen lassen. Darüber hinaus kommen auch mit thermoplastischen Kunststoffen beschichtete Papiere in Frage. Spezielle Einfärbungen oder Beschichtungen der Folien können dem eingeschlossenen Produkt Licht-, Feuchtigkeits- oder Oxidationsschutz geben. Die Folien können zur näheren Identifizierung des Produktes bedruckt oder in anderer Weise markiert sein. Der Begriff Folien soll alle Materialien erfassen, die dem vorgenannten Zweck der Ausbildung von Hohlformen aus Folien dienen können, also neben Kunststoff-Folien auch Metallfolien, beschichtete Papier- und Kartonmaterialien usw.

Bei der Herstellung der Hohlformen kann in vielfältiger Weise vorgegangen werden. Die nachfolgend beschriebenen Verfahren sollen demzufolge nur bevorzugte Beispiele angeben. Mit Hilfe von Formwerkzeugen können aus jeweils zwei Folien symmetrische oder asymmetrische Halbhohlformen gezogen werden, die an ihren Rändern dicht verschweißt sein müssen. Im oberen Teil der Hohlform bleibt zunächst eine trichterförmige Öffnung bestehen, durch die die erwähnte Rezepturmasse eingebracht wird. Die Einfüllöffnung wird nach dem Füllvorgang in üblicher Weise abgesiegelt. Es entsteht eine rundum dichte Packung. Das Formen und das Füllen können jeweils so durchgeführt werden, daß die einzelnen Hohlformen zu mehreren "Streifen" zusammenhängen, von denen sich der Verbraucher eine Einzeldosis abtrennen kann. Das in die Hohlform eingebrachte Produkt wird durch Auseinanderreißen der Folien freigelegt.

Von besonderem Vorteil kann es im Einzelfall sein, wenn die Folien einen kleinen, nicht versiegelten Bereich aufweisen, in dem eine Folie die andere um einige Millimeter überragt. Diese Überlappung erleichtert die Handhabung beim Auseinandertrennen der Folien.

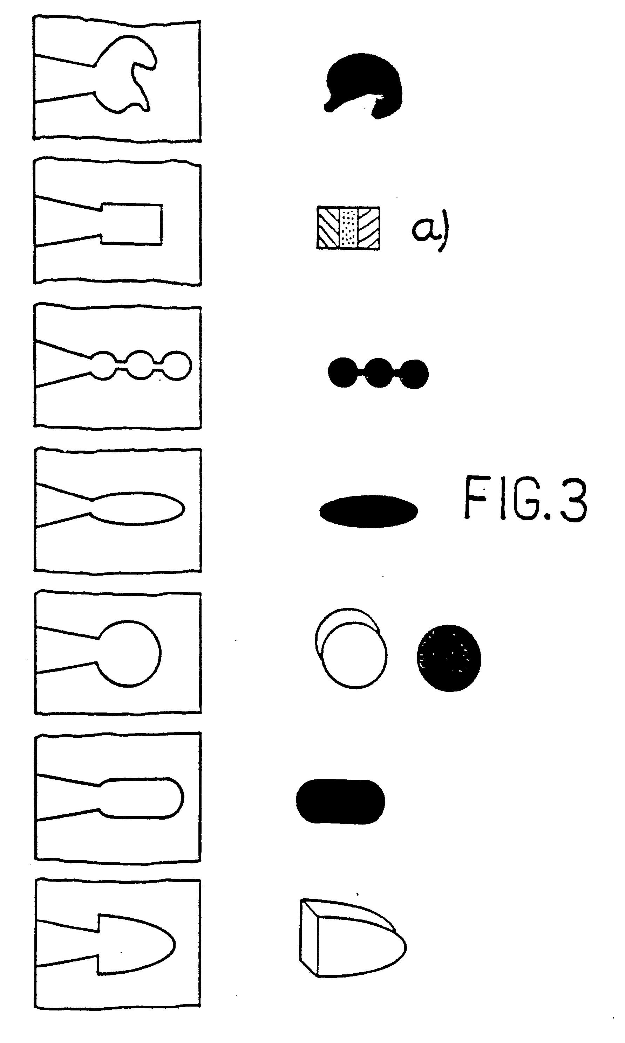

Die einzelnen Hohlformen lassen sich z. B. auch mit Einschnürungen versehen, die eine spätere Teilung, d. h. Portionierung des Produktes erlauben, z. B. für eine Kinderdosis oder für Teilstücke eines Vollkörpers als Gabe "3 x täglich" (Fig. 3).

Die Figur 1 gibt einige Beispiele für verschiedene erfindungsgemäß erhaltene Darreichungsformen bzw. deren Hohlformen zur Erläuterung.

Das bisher beschriebene erfindungsgemäoe Verfahren zur Herstellung von Formlingen ohne werkzeugabhängige Formgebung und das Befüllen von Durchdrückpackungen und vorgeformten Hohlformen soll nun ergänzt werden durch die Beschreibung der Substanzen und Massen, die sich als Rezepturmassen völlig von den bekannten Suppositorienrezepturen oder den in der DE-A- 27 10 307 angedeuteten Massen unterscheiden. Weiterhin sollen maßgebliche Vorteile gegenüber bisherigen Darreichungsformen erläutert werden.

Während die Füllgüter für das Befüllen von Suppositorienformen oder Formen entsprechend der DE-A- 27 10 307 relativ niedrig-schmelzende Fettkörper bevorzugen, können geeignete Substanzen für Basisrezepturen nach dem erfindungsgemäßen Verfahren z. B. Rohstoffe sein, die zu den Gelbildnern gehören. Diese bilden mit Wasser, anderen Lösemitteln und/oder Weichmachern in der Wärme Sole. Das Lösen der Gelbildner kann bei Temperaturen über 100° C erfolgen. In die abgekühlte Lösung können weitere Hilfsstoffe und Wirkstoffe eingearbeitet werden, zumeist durch Kneten. Die Viskosität erreicht Werte zwischen 50 000 und 800 000 mPA's, bei Raumtemperatur.

Die mit Hilfe der beschriebenen Dosiergeräte erhaltenen Körper sind elastisch, bruchsicher und geeignet zum Schlucken, Lutschen, Kauen oder zur bukkalen oder perlingualen Anwendung. Sie sind derart angelegt, daß sie nicht wie die Fettmassen bei Körpertemperatur schmelzen, sondern gezielt mehr oder weniger schnell in den Körperflüssigkeiten zerfallen oder sich darin lösen. Sofern Wasser zum Ansetzen verwendet wurde, kann dieses entweder in den Rezepturen verbleiben oder man läßt die Körper, z. B. in Klimaschränken, auf einen gewissen Feuchtigkeitsgehalt auftrocknen, womit Härte und Elastizität gesteuert werden können.

In Abhängigkeit von der Löslichkeit der Hilfs- und Wirkstoffe kommt es zu unterschiedlichen, steuerbaren Freisetzungen der Wirkstoffe, wobei für die Hilfsstoffe nach Art und Menge eine große Variationsbreite besteht. Auf diese Weise lassen sich leicht Produkte mit ausgeprägter Retardwirkung herstellen.

Eine besondere erfindungsgemäß erhältliche Darreichungsform stellen solche Produkte dar, die magensaftresistent sein sollen. Durch Einarbeitung von sich im Magensaft nicht lösenden Substanzen oder Substanzgemischen in die Basisrezepturen oder durch ausschließliche Verwendung solcher Substanzen oder Substanzgemische gelangt man zu Produkten, die dem Test der Arzneibücher auf Magensaftresistenz standhalten.

Die erfindungsgemäßen Darreichungsformen gestatten es auch, Produkte herzustellen, bei denen sich Initialdosis und Retardform in einer Dosis befinden. Als besonderes Beispiel hierfür wird das Schlafmittel Chloralhydrat angeführt, bei dem es sich um ein schnell, aber auch kurz wirkendes Hypnoticum handelt. Um dem Patienten die zwei- oder mehrmalige Einnahme dieses Medikamentes während des Abends oder in der Nacht zu ersparen und um ein gleichmäßig tiefes Durchschlafen zu ermöglichen, ist es erwünscht, mit nur einer abendlichen Gabe eine Schlafperiode von etwa 8 Std zu gewährleisten, ohne daß es am Morgen zu einem hangover kommt. Erfindungsgemäß werden daher zwei oder drei Chloralhydrat enthaltende Formulierungen mit unterschiedlicher Freisetzung rezeptiert. Diese werden der Reihe nach übereinander in die Hohlform gespritzt. Das Produkt sieht dann wie Fig. 3a zeigt, geschichtet aus, wobei die Schichtung, z.B. durch Anfärben der einzelnen Rezepturen, noch verdeutlicht werden kann. Eine Weiterführung dieses Gedankens ist die Verarbeitung an sich unverträglicher Substanzkombinationen, wie sie - beispielhaft zur Veranschaulichung hier angeführt - bei Vitamin-Mineral-Mischungen vorliegen kann. Mit der ersten Spritzung wird die Vitamin-Rezeptur eingebracht, mit der zweiten Spritzung eine Neutral-Rezeptur als Schutzschicht und mit der dritten Spritzung schließlich die Mineral-Rezeptur. Dabei ergibt sich eine analoge Schichtung, wie in Fig. 3a dargestellt. Es ist selbstverständlich, daß die Basis-Rezepturen in diesem Falle miteinander verschmelzen müssen, um einen einzigen massiven Vollkörper zu ergeben. Weiter oben wurde bereits darauf hingewiesen, daß es keine Schwierigkeiten macht, die einzelnen Spritzköpfe des Abfüllsystems für solche Spritzfolgen zu arrangieren. Es versteht sich von selbst, daß die zu dem obigen Beispiel gemachten konkreten Angaben ganz allgemein zu werten sind, d.h. zur Herstellung von erfindungsgemäß geschichteten Darreichungsformen.

Für die bukkale oder sublinguale Verabreichung geeignete Produkte lassen sich nach dem Grundprinzip vorliegender Erfindung in mannigfacher Form und Zusammensetzung herstellen. So können beispielsweise kugelförmige Körper mit kleiner Oberfläche oder langgezogene Streifen mit großer Oberfläche geformt werden. Je nach gewünschter Verweildauer im Mund können leicht lösliche oder nahezu unlösliche Substanzen oder Gemische zur Ausbildung des Arzneikörpers herangezogen werden. Ein großer Vorteil der Erfindung liegt darin, daß Schleimstoffe verarbeitet werden können, die lange an der Mundschleimhaut haften und somit einen engen Kontakt des zu applizierenden Wirkstoffes mit den Schleimhäuten ermöglichen.

Eine weitere Möglichkeit der Nutzung vorliegender Erfindung ist die Herstellung von Kau- und Lutschkörpern, deren Löslichkeit zwischen schnell und "kaugummiähnlich" schwanken kann. Es lassen sich z.B. Stoffe einbetten, die als Mund- und Rachendesinfizientien oder Adstringentien bekannt sind oder als Atemerfrischer. Weiterhin können in die Basisrezeptur unlösliche Substanzen eingebettet werden, die einen gewissen Scheuereffekt haben und beim Kauen der Zahnreinigung dienen. Kaubare Antacida, die sich als viskose Suspensionen filmbildend an die Magenschleimhaut anschmiegen, sind ein weiteres Einsatzgebiet im Rahmen der großen Anwendungsbreite der erfindungsgemäß erhältlichen Kaukörper. Bei diesem Beispiel wird die Überlegenheit der Darreichungsformen der vorliegenden Erfindung besonders deutlich. Bisher verwendete man als kaubare Antacida Tabletten, die durch den Kauvorgang krümelig zerfallen. Die einzelnen Partikel reizen Rachen und Schlund, was zu unangenehmem Räuspern Anlaß gibt. Bei den erfindungsgemäßen Formen bildet sich im Mund eine viskose Suspension, die ohne Reizung der Schleimhäute heruntergeschluckt werden kann.

Erfindungsgemäß können auch Emulsionen, z.B. vom W/O- oder O/W-Typ, abgefüllt werden, die in Gegenwart von Körpersäften zu feindispersen Tropfen zerfallen und damit die Resorption von Ölen und Fetten und gegebenenfalls darin gelösten Stoffen erleichtern. Natürlich müssen diese Emulsionen unterhalb etwa 40°C erstarren, um sie aus den Hohlformen nehmen zu können.

Die Erfindung erlaubt es des weiteren, den herzustellenden Dosisformen auch Stoffe beizugeben, die zur Wahrung oder zur Erzielung eines speziellen pH- oder HLB-Wertes erforderlich sind. Die Substanzen können z.B. Puffergemische oder Emulgatoren mit bestimmtem HLB-Wert sein.

Im Gegensatz zu Gelatinekapseln, bei denen die Befüllung mit Enzymen problematisch ist, lassen sich diese in den erfindungsgemäß erhältlichen Produkten leicht verarbeiten, da weder Hüllen noch Hilfsstoffe verwendet werden müssen, die durch die Enzyme angegriffen werden und damit die Enzymaktivität schmälern.

Ein weiterer Vorteil der vorliegenden Erfindung ist darin zu sehen, daß die Verarbeitung öliger Substanzen, die üblicherweise den Weichgelatinekapseln vorbehalten ist, leicht dadurch möglich ist, daß das Öl auf einen Trägerstoff aufgezogen wird. Dieses Gemisch wird dann zusammen mit einem Gelbildner zu Formen gespritzt, die keine Neigung zum "Ausbluten" oder Lecken zeigen. Auf diese Weise lassen sich auch wässrige Lösungen verarbeiten, ein für Hart- und Weichkapselfüllungen nicht denkbarer Vorgang.

Als Stoffe und Repräsentanten von Stoffklassen bzw. als Trägermassen, die für die aufgezeigten Verfahren und Anwendungsgebiete eingesetzt werden können, kommen insbesondere in Frage: Albumine, Gelatine, Kasein, Pflanzenproteine, Zein, Lecithine, Agar-Agar, Gummi arabicum, Pektine, Alginate, Xanthan, natürliche und modifizierte Stärken, Maltodextrin, Methylcellulose, Ethylcellulose, Celluloseäther-polysaccharide, Carboxymethylcellulosen, verätherte Carboxymethylcellulosen, Hydroxypropylcellulose, Hydroxypropylmethylcellulosephthalat, Celluloseacetatphthalat, Polyvinylacetat, Polyvinylacetatphthalat, Polyvinylalkohol, Polyvinylpyrrolidon, Polyacrylsäure, Polymere aus Methacrylsäure und Methacrylsäureestern, Polyethylenglykole, Poloxalkole, Triglyceride, Partialglyceridgemische, ethoxylierte Partialglyceride, langkettige Fettsäuren und ihre Salze, Silicone, kolloidale Kieselsäure, Bentonite u.a.

Zu den als Weichmacher verwendbaren Substanzen gehören z.B. Glycerol, Propylenglykol, Polyethylenglykole, Acetyltributylcitrat, Triacetin, Dibutyltartrat, Dibutylphthalat, Sorbit, Sorbitangemische, Glucosesirup u.a.

Der Vollständigkeit halber soll erwähnt werden, daß den erfindungsgemäß erhältlichen Produkten auch Zuschlagstoffe, wie z.B. Opakifizierungsmittel, Farbstoffe, Aromen, Süßstoffe, Konservierungsstoffe und dergleichen, beigegeben werden können.

Für die Erfindung ist es wichtig, daß die oben genannten Materialien zur Bereitung einer Rezepturmasse so abgestimmt werden, daß die Haftung zwischen der erstarrten Rezepturmasse und der inneren Oberfläche der Hohlform auf ein Minimum zurückgeführt wird. In der Mehrzahl der Fälle lassen die geläufigen thermoplastischen Kunststoffe sowie Trägermassen bzw. Rezepturmassen eine saubere Trennung zu. Sollte das in einzelnen Fällen dennoch nicht möglich sein, dann bietet die Erfindung hierfür weitergehende vorteilhafte Hilfsmittel an, auf die nachfolgend eingegangen wird.

Falls es erforderlich ist, lassen sich im Rahmen der Erfindung die Hohlformen vor dem Befüllen mit der Rezepturmasse mit Trennschichten versehen, um ein Kleben oder Haften zwischen Folienoberfläche und Rezepturmasse zu verhindern bzw. auf ein Mindestmaß zurückzuführen. Dabei wird es bevorzugt, daß die Paarungen Rezepturmasse/Trennmittel die Affinitätspaarung lipophil/lipophob oder hydrophil/hydrophob aufweisen. Bevorzugte Substanzen für diesen Zweck sind z.B. Talkum, Stärken und dergleichen. Es ist aber auch möglich, Trennmittel, wie etwa Öle, Fette oder Silicone, in der Hohlform zu vernebeln oder zu verdüsen. Schließlich können derartige Substanzen auch mit bürstenähnlichen, rotierenden Werkzeugen, die sich innerhalb der Hohlform spreizen, auf deren Innenwandung aufgebracht werden. Für Tiefziehpackungen gilt Entsprechendes.

Von Vorteil kann es sein, daß das Trennmittel bereits vor dem Ausformen der Hohlform auf eine Seite der Ausgangsfolie aufgebracht wird, wobei diese präparierte Seite später die innere Oberfläche der Hohlform darstellt.

Mit den oben beschriebenen Beschichtungstechniken lassen sich aber auch solche Beschichtungen auf die Innenwand der Hohlform legen, die nicht als Trennmedium fungieren, sondern als Trägerschicht für Gleitmittel oder für Farbstoffe, Aromen, Süßstoffe, Konservierungsmittel und dergleichen dienen und u.a. die Applikation des Produktes günstig beeinflussen. Diese Trägerschicht verbindet sich mit der eingebrachten Rezepturmasse und legt sich wie ein Mantel um die Vollkörper. Auf diese Weise wird es erreicht, daß nur die äußere Schicht des Produktes z. B. angefärbt oder aromatisiert wird, was zu einer erheblichen Reduzierung der Korrigentien führt, die sonst in wesentlich größerer Menge im gesamten Füllkörper homogen verteilt sein müßten.

Wie ersichtlich, bietet die Erfindung vielfältige Vorteile. So ermöglicht sie die kostensparende und umweltbewußte Herstellung von oral applizierbaren Darreichungsformen, wobei die eingangs aufgezeigten Nachteile bei der Bereitung wichtiger Darreichungsformen weitgehend vermieden werden, insbesondere preiswerte Produkte angeboten werden können, die sich ohne Mühe in großer Stückzahl als Massenware herstellen lassen, wobei eine nahezu unbegrenzte Vielfalt unterschiedlichster Rezepturen für sehr viele Indikationsgebiete eingesetzt werden kann. Dabei lassen sich auch an sich unverträgliche Stoffe in einer einzigen Darreichungsform präsentieren, ohne daß sie miteinander reagieren. Auch können die Löslichkeit eines Wirkstoffs beeinflussende Hilfsstoffe, wie z.B. feste und flüssige Stoffe, nebeneinander und in großer Zahl verarbeitet werden, so daß gezielte Wirkstoff-Freisetzungen zustandekommen.

Aus der Beschreibung des erfindungsgemäßen Verfahrens wird es deutlich, daß der für viele Darreichungsformen obligatorische Formgebungsprozeß entfallen kann, weil die Verpackung gleichzeitig das formgebende Element darstellt. Daraus folgt als wichtige Erkenntnis, daß es keinerlei Verpackungsprobleme in Bezug auf Zuführung der Objekte zu den Verpackungsmaschinen oder in Bezug auf weitstreuende Maßtoleranzen geben kann, wie sie bis heute etwa bei Weichgelatinekapseln nicht zu vermeiden sind. Die so sehr gefürchtete Untermischung von "Fremdkörpern", etwa Fremddragées oder Fremdkapseln, wie sie in bisherigen Prozessen bei der Herstellung oder während der Verpackung auftreten kann, ist gänzlich ausgeschlossen, da das Produkt direkt und in flüssigen Zustand in die Verpackung eingebracht und die Einfüllöffnung versiegelt bzw. abgesiegelt wird. Durch spezielle Gestaltung von Folie, Form und Inhalt kann den Produkten ein unverwechelbares Image gegeben werden.

Darüber hinaus erlaubt es die Erfindung, eine außergewöhnlich große Zahl von Substanzen zu verarbeiten, sei es als Hilfs- oder als Wirkstoffe, seien es feste und flüssige Stoffe nebeneinander. Diese Vielfalt kann zu Produkten mit gesteuerter Wirkstoff-Freisetzung in weiten Grenzen führen, wie auch zu magensaftresistenten Präparaten. So lassen sich auch Kau- und Lutschkörper herstellen.

Viele der neuartigen Produkte, soweit sie nicht als Lutschkörper oder magensaftresistente Präparate konzipiert sind, können, da sie zumeist elastisch sind, zerbissen und in kleinen Stücken - auch ohne daß Flüssigkeit nachgetrunken werden muß - geschluckt werden, was Patienten mit Schluckbeschwerden oder Widerwillen gegen Tabletteneinnahme als Erleichterung empfinden.

Auch unter wirtschaftlichen Gesichtspunkten bietet die Erfindung wesentliche Vorteile. So entfällt der separate Schritt der Formgebung, was allein schon eine entscheidende Verbilligung gegenüber den bisher gebräuchlichen Verfahren darstellt. Der hohe Ausstoß an gefüllten und damit zugleich verpackten Formen pro Zeiteinheit und die leicht zu installierenden on-line Inprozeßkontrollen stellen eine weitere kostensparende Rationalisierung dar. Soweit sich das erfindungsgemäße Verfahren als Ersatz bisher üblicher Kapselfertigungsmethoden eignet, entfallen auch die Kosten für aufwendige Klimaanlagen und Anlagen zur Rückgewinnung von Lösungsmitteln samt Folgekosten. Da - wiederum im Gegensatz zur Kapselfertigung - kein Rohstoff vergeudet wird (Anteil Netz bei der Weichkapselfertigung ca. 50% und Anteil trimmings bei der Hartkapselfertigung ca. 30% der eingesetzten Gelatine) ist auch hier eine deutliche Ersparnis gegeben. Schließlich ist das erfindungsgemäße Verfahren ohne Umweltbelastung durchführbar.

Die nachfolgend aufgeführten Beispiele sollen die Erfindung näher erläutern.

Die verschiedenartigsten Trägerstoffe können in mannigfacher Weise zur Erzielung der unterschiedlichen Verwendungszwecke miteinander kombiniert werden. Die Vielfalt der Möglichkeiten des erfindungsgemäßen Verfahrens kann durch die Beispiele nur angedeutet werden.

Beispiel 1

| | |
|---|---|
| Gelatine | 120 g |
| Sorbitlösung (70 %ig) | 180 g |
| Wasser | 373 g |
| Eiweißhydrolysat, wasserlöslich | 600 g |
| Sorbinsäure | 5 g |
| Zuckercouleur | 12 g |
| Fleischextrakt-Aroma | 10 g |

1.300 g

In der Lösung von Sorbinsäure in Wasser wird die Gelatine bei 70° C unter Vakuum gelöst. Nacheinander werden in diese Lösung Aroma, Zuckercouleur, Sorbitlösung und Eiweißhydrolysat eingearbeitet. Die Mischung wird in der Wärme entlüftet. Die Masse wird bei 56° C und 48 bar auf eine 10° C gekühlte Unterlage zu Häufchen von je 1 300 mg gespritzt. Es wird bei 20 % r. F. und Raumtemperatur so lange getrocknet, bis jede Einzeldosis 1 090 mg wiegt.

Beispiel 2

| | |
|---|---|
| Abgebaute, veresterte Stärke (Emgum R) | 120 g |
| Oxidierte Weizenstärke (Emox R) | 180 g |
| Zucker | 50 g |
| Glucosesirup 75 % | 175 g |
| Wasser | 200 g |
| Chlorhexidin | 6 g |
| Extractum Arnicae plv. subt. | 10 g |
| Zitronensäure-Na-citrat 1:1 | 20 g |
| Ingwer-Aroma | 7 g |
| | 768 g |

Zucker, Glycosesirup und Wasser werden bis zum Erhalt einer klaren Lösung auf 110° C erhitzt. In die auf 70° C abgekühlte Lösung werden unter kräftigem Rühren beide Stärkesorten eingetragen. Die Mischung wird kurz auf 90° C erwärmt. Nach erneutem Abkühlen auf 70° C werden die übrigen Substanzen in aufsteigender Reihenfolge ihrer Gewichte homogen eingearbeitet. Die Masse wird bei 70° C und 90 bar mit einer Dosierkopftemperatur von 85° C in Blister mit tablettenförmiger Mulde gespritzt. Jede Dosis enthält 750 mg. Die Absiegelung erfolgt mit einer peel-off-Folie.

Beispiel 3

| | |
|---|---|
| Herzglycosid | 0,5 g |
| Ethoxyliertes Partialglycerid-Gemisch natürlicher, gesättigter geradzahliger Pflanzenfettsäuren | 100,0 g |
| (Softigen 767 R) | |
| Polyoxyethylen-polyoxypropylen-Polymer (Poloxalcol) | 700,0 g |
| Polyethylenglycol 400 (PEG) | 199,5 g |
| | 1.000,0 g |

In das auf 60° C erwärmte PEG wird unter mäßigem Rühren das geschmolzene, auf 60° C gehaltene Poloxalcol eingetragen. Die auf 45° C erwärmte Lösung des Herzglycosids im Softigen wird homogen in die erste Lösung eingerührt. Die auf 45° C abgekühlte Masse wird zu je 200 mg mit 22 bar in ovalförmige, auf 10° C gekühlte Hohlformen eingespritzt. Die gefüllten Formen können sofort versiegelt werden.

Beispiel 4

| | |
|---|---|
| Gelatine | 240 g |
| Hydroxypropyl-methyl-cellulose-phthalat (HP 55) | 90 g |
| Wasser | 389 g |
| Ammoniak 25 % | 21 g |
| Glycerol 85 % | 260 g |
| | 1.000 g |

Die homogene Pulvermischung aus Gelatine und HP 55 wird schnell und unter Rühren in die Mischung aus Wasser und Ammoniak eingetragen. Es ist wichtig, daß alle Pulverpartikel gleichmäßig benetzt sind. Nach einer Quellzeit von 12 min bei Raumtemperatur wird die krümelige Masse im Vakuum bei 90° C geschmolzen. In die so erhaltene zähfließende Masse wird das auf 40° C erwärmte Glycerol eingerührt. Die abgekühlte Masse stellt einen Grundkörper für eine magensaft-resistente Darreichungsform dar. Durch Anfärben der Masse bei der Herstellung, z. B. mit Erythrosin, läßt sich leicht zeigen, daß dosierte Portionen 2 Stunden gegen künstlichen Magensaft (0,1n HCl) resistent sind, ohne auszubluten, während sie sich nach Überführung in eine Pufferlösung (0,2 m $Na_2HPO_4$-Lösung 773 ml + 0,1 m Zitronensäure-Lösung 227 ml; pH 6,8) innerhalb 26 min gleichmäßig und rückstandslos lösen.

Durch Einarbeiten von Füllmaterialien, z. B. Maisstärke, in die Grundrezeptur bleibt die Magensaftresistenz erhalten, während die Löslichkeit in künstlichem Darmsaft steuerbar auf mehrere Stunden verlängert werden kann.

Beispiel 5

| | |
|---|---|
| Gelatine | 240 g |
| Celluloseacetat-phthalat (CAP) | 90 g |
| Wasser | 410 g |
| Glycerol 85 % | 260 g |
| | 1.000 g |

Die Herstellung dieser Rezeptur erfolgt analog Beispiel 4. Das Verhalten dosierter Portionen entspricht den Körpern aus Beispiel 4.

Beispiel 6

| | |
|---|---|
| Polyminylpyrrolidon (PVP) (Kollidon 30) | 50 g |
| Oxidierte Stärke (Emox 170$^R$) | 250 g |
| Glycerol 85 % | 250 g |
| Wasser | 230 g |
| Maltodextrin | 480 g |
| Diphenhydramin-HCl | 10 g |
| Fenchel-Honig-Aroma | 10 g |
| | 1.280 g |

Wasser und Glycerol werden gemischt. In die kalte Lösung wird das PVP eingetragen und bis zur vollständigen Lösung gerührt. In der nun auf 95° C erwärmten Lösung wird zunächst unter Rühren das Maltodextrin gelöst, dann wird bei ebenfalls 95° C die Stärke eingerührt. In die auf 75° C abgekühlte Mischung werden das Diphenhydramin und das Aroma homogen eingearbeitet. Die Masse wird bei 65 - 70° C und 95 bar zu je 1.280 mg in Blister abgefüllt, die unmittelbar nach dem Erstarren der Masse mit peel-off-Folie abgesiegelt werden.

## Ansprüche

1. Verfahren zur Herstellung einzeln dosierter Darreichungsformen, wobei eine bei erhöhter Temperatur plastische, Wirkstoffe und übliche Additive enthaltende, auf einer Sol-/Gel-Beziehung beruhende Rezepturmasse eines oberhalb der Körpertemperatur liegenden Erweichungsbereiches auf über Körpertemperatur erwärmt und mit einer Viskosität von mehr als 3000 mPa.s unter Druckeinwirkung auf eine ruhende oder bewegte Unterlage gebracht oder in aus Folien hergestellte Blister oder Hohlformen eingebracht und die Rezepturmasse hierdurch und unter Abkühlen in eine Darreichungsform überführt wird, dadurch **gekennzeichnet,** daß die zu dosierende warme Rezepturmasse aus einem Aufheiztank in ein mit hohem Druck arbeitendes heizbares Dosiergerät geführt wird, das ansteuerbare Ventile besitzt, gegen die Kolben- oder Zahnradpumpen einen gleichmäßig hohen Druck aufbauen, und daß durch zeitlich genau begrenztes Öffnen der Ventile eine gleichbleibende Menge der zu dosierenden warmen Rezepturmasse auf die Unterlage oder in die Blister oder Hohlformen ausgetragen wird.

2. Verfahren nach Anspruch 1, dadurch gekennzeichnet, daß die zu dosierende warme Rezepturmasse mit Hilfe von in den Aufheiztank ragenden Pumpen unter regulierbarem Druck und Bypass-Steuerung in heizbare Hochdruckschläuche gebracht wird.

3. Verfahren nach Anspruch 2, dadurch gekennzeichnet, daß die heizbaren Hochdruckschläuche in heizbare Dosierköpfe münden, die mit federgelagerten Kugelventilen und Düsen ausgerüstet sind.

4. Verfahren nach mindestens einem der Ansprüche 1 bis 3, dadurch gekennzeichnet, daß mit Hilfe gesteuerter Preßluft nach Millisekunden gerechnete Ventilöffnungs- und -schließspiele bewirkt werden, wobei je nach anstehendem Druck vor dem Ventil bei gleichen Drucken und Öffnungszeiten gleich viel Masse ausgetragen wird.

5. Verfahren nach einem der Ansprüche 1 bis 4, dadurch gekennzeichnet, daß die warme Rezepturmasse auf eine runde oder bewegte Unterlage aufgebracht wird, wobei ohne Verwendung formgebender Elemente allein aufgrund der Temperatur und der Viskosität der Rezepturmasse und eventuell ausgeführter Bewegungen des Dosierkopfes die gewünschte Darreichungsform entsteht.

6. Verfahren nach mindestens einem der Ansprüche 1 bis 4, dadurch gekennzeichnet, daß auf die innere Oberfläche der Hohlform oder des Blisters ein die Haftung herabsetzendes Trennmittel aufgebracht wird.

7. Verfahren nach mindestens einem der Ansprüche 1 bis 4, dadurch gekennzeichnet, daß die Folien zur Ausbildung der Hohlform einen nicht gesiegelten Bereich aufweisen, in dem eine Folie die andere um einige Millimeter überragt.

8. Verfahren nach Anspruch 7, dadurch gekennzeichnet, daß die Rezepturmasse und das Trennmittel entgegengesetzte Affinität aufweisen.

9. Verfahren nach Anspruch 6, dadurch gekennzeichnet, daß im Falle einer lipophilen bzw. hydrophoben Rezepturmasse vor Einbringen derselben Wasser in die Hohlform oder in den Blister eingenebelt wird.

10. Verfahren nach Anspruch 6, dadurch gekennzeichnet, daß im Falle hydrophiler Rezepturmassen die innere Oberfläche der Hohlform oder des Blisters mit hydrophoben Ölen beschichtet wird.

11. Verfahren nach einem der Ansprüche 1 bis 4 und 6 bis 10, dadurch gekennzeichnet, daß die Hohlform oder der Blister aus einem thermoplastischen Kunststoff, aus mit thermoplastischem Kunststoff beschichtetem Aluminium oder aus kunststoffkaschiertem Papier besteht.

12. Verfahren nach einem der Ansprüche 1 bis 4 und 5 bis 11, dadurch gekennzeichnet, daß das Trennmittel bereits vor dem Ausformen der Hohlform oder des Blisters auf eine Seite der zu formenden Folie aufgebracht wird, wobei diese Seite später die innere Seite der Hohlform bzw. des Blisters darstellt.

13. Verfahren nach einem der Ansprüche 1 bis 4 und 6 bis 12, dadurch gekennzeichnet, daß auf die innere Obrefläche der Hohlform oder des Blisters die Applikation und/oder die Akzeptanz der fertigen Rezepturmasse günstig beeinflussende Mittel aufgetragen werden.

14. Verfahren nach Anspruch 13, dadurch gekennzeichnet, daß die Mittel Farbstoffe, Aromen, Süßstoffe, Konservierungsstoffe und/oder Gleitmittel darstellen.

15. Verfahren nach einem der Ansprüche 1 bis 14, dadurch gekennzeichnet, daß eine eine steuerbare Retardwirkung zeigende Rezepturmasse, eine Magensaftresistenz zeigende Rezepturmasse oder eine der bukkalen oder sublingualen Anwendung dienende Rezepturmasse auf eine Unterlage gebracht oder in die Hohlform oder den Blister eingebracht wird.

16. Verfahren nach einem der Ansprüche 1 bis 15, dadurch gekennzeichnet, daß ein Kau- oder Lutschkörper aus der Rezepturmasse hergestellt wird.

17. Verfahren nach einem der Ansprüche 1 bis 4 und 6 bis 16, dadurch gekennzeichnet, daß die Hohlformen oder Blister nacheinander mit unterschiedlichen Rezepturmassen gefüllt werden.

18. Verfahren nach Anspruch 17, dadurch gekennzeichnet, daß die eine Schicht die Initialdosis enthält und die andere Schicht die Retardform darstellt.

19. Einzeln dosierte Darreichungsformen mit einer thermoplastischen Rezepturmasse eines oberhalb der Körpertemperatur liegenden Erweichungspunktes, **erhältlich** durch das Verfahren nach mindestens einem der Ansprüche 1 bis 18.

**Claims**

1. A process for producing individually dosed administration forms, wherein a formulation material containing conventional additives and active substances which are plastic at elevated temperature and of a sol/gel ratio having a softening range above body temperature is heated above body temperature and, at a viscosity of more than 3000 mPa.s and under the action of pressure, is placed on a stationary or moving substrate or is introduced into blisters or cavities produced from films with the result that the formulation material is transformed, accompanied by cooling, into an administration form, characterised in that the heated formulation material to be made into doses is taken from a heating tank and passed into heatable dosing equipment operating at high pressure which has controllable valves against which the piston pumps or gear pumps build up a uniform high pressure, and that through precisely-timed limited opening of the valves an always-constant quantity of the heated formulation material to be made into doses is discharged onto the substrate or is introduced into the blisters or cavities.

2. A process according to claim 1, characterised in that the heated formulation material to be made into doses is brought into heatable high pressure hoses with the aid of pumps projecting into the heating tank and under regulatable pressure and by-pass control.

3. A process according to claim 2, characterised in that the heatable high pressure hoses issue into heatable dosing heads which are provided with spring-mounted ball valves and nozzles.

4. A process according to any one of claims 1-3, characterised in that, with the aid of controlled compressed-air valve-opening and valve-closing cycles calculated in milliseconds, the same amount of material is always discharged for the same pressures and opening times as a function of the pressure upstream of the valve.

5. A process according to any one of claims 1-4, characterised is that the heated formulation material is applied to a stationary or moving substrate, the desired administration form being obtained without the use of shaping elements and solely on the basis of the temperature and viscosity of the formulation material and possibly performed movements of the dosing head.

6. A process according to at least one of claims 1-4, characterised in that an adhesion-reducing separating agent is applied to the inner surface of the cavity or blister.

7. A process according to at least one of claims 1-4, characterised in that the films for forming the cavity have an unsealed area, with one film projecting over the other by a few millimetres.

8. A process according to claim 6, characterised in that the formulation material and the separating agent have opposite affinities.

9. A process according to claim 6, characterised in that, in the case of a lipophilic or hydrophobic formulation material, water is atomised into the cavity or blister prior to the introduction of the formulation material.

10. A process according to claim 6, characterised in that, in the case of hydrophilic formulation materials, the inner surface of the cavity or blister is coated with hydrophobic oils.

11. A process according to any one of claims 1-4 and 6-10, characterised in that the cavity or blister is made from a thermoplastic material, or from aluminium coated with thermoplastic material, or from synthetic plastic-lined paper.

12. A process according to any one of claims 1-4 and 6-11, characterised in that the separating agent is applied to one side of the film to be shaped prior to the removal of the cavity or blister, the said side of the film subsequently forming the inner surface of the cavity or blister.

13. A process according to any one of claims 1-4 and 6-12, characterised in that agents favourably influencing the application and/or acceptance of the finished formulation material are applied to the inner surface of the cavity or blister.

14. A process according to claim 13, characterised in that the agents are dyes, flavours, sweeteners, preservatives and/or lubricants.

**15.** A process according to any one of claims 1-14, characterised in that a formulation material having a controllable retarding action, a formulation material having a gastric juice resistance, or a formulation material for buccal or sublingual application is applied to a substrate or introduced into the cavity or blister.

**16.** A process according to any one of claims 1-15, characterised in that a chewing or sucking article is produced from the formulation material.

**17.** A process according to any one of claims 1-4 and 6-16, characterised in that the cavities or blisters are successively filled with different formulation materials.

**18.** A process according to claim 17, characterised in that one coating contains the initial dose and the other coating the retard form.

**19.** Individually-dosed administration forms with a thermoplastic formulation material having a softening point above body temperature obtainable through the process according to at least one of claims 1-18.

## Revendications

**1.** Procédé de préparation de formes propre à l'administration de doses individuelles, en chauffant une masse de formulation, plastique à température élevée, renfermant des substances actives et des additifs usuels s'appuyant sur une relation sol/gel, d'un domaine de ramollissement supérieur à la température du corps, et en l'amenant avec une viscosité de plus de 3.000 mPa.s sous l'action de la pression sur un substrat au repos ou en mouvement ou en l'introduisant dans les ampoules (blisters) ou formes creuses obtenues à partir de feuilles et en faisant passer, ainsi et avec refroidissement, la masse de formulation sous une forme administrable, caractérisé en ce qu'on transfère la masse de formulation chaude à doser d'une cuve de chauffage à un appareil de dosage chauffable fonctionnant sous haute pression, qui comporte des soupapes commandées contre lesquelles des pompes à pistons ou à engrenages établissent une pression de niveau uniforme, et en ce que par une ouverture des soupapes dans des intervalles de temps délimités avec précision, on fait passer une quantité constante de la masse de formulation chaude à doser sur le substrat ou dans les ampoules ou formes creuses.

**2.** Procédé selon la revendication (1), caractérisé en ce qu'on fait passer la masse de formulation chaude à doser à l'aide de pompes pénétrant dans la cuve de chauffage, sous une pression régulatrice et avec commande de dérivation dans des tuyaux à haute pression chauffables.

**3.** Procédé selon la revendication (2), caractérisé en ce que les tuyaux sous haute pression chauffables débouchent dans des têtes de dosage chauffables qui sont pourvues de soupapes à boulets montées sur ressorts et de buses.

**4.** Procédé selon l'une au moins des revendications 1 à 3, caractérisé en ce qu'à l'aide d'air comprimé réglé, on provoque des ouvertures et des fermetures de soupape calculés en millisecondes, et selon la pression régnant, devant, la soupape, une même quantité de masse est débitée pour des pressions égales et des durées d'ouverture égales.

**5.** Procédé selon l'une quelconque des revendications 1 à 4, caractérisé en ce que la masse de formulation chaude est appliquée à un substrat au repos ou en mouvement, en obtenant la forme administrable souhaitée sans utiliser d'éléments de mise en forme, rien que sur la base de la température et de la viscosité de la masse de formulation et de mouvements éventuellement effectués de la tête de dosage.

**6.** Procédé selon l'une au moins des revendications 1 à 4, caractérisé en ce qu'on applique à la surface intérieure de la forme creuse ou de l'ampoule un agent de séparation réduisant l'adhérence.

**7.** Procédé selon l'une au moins des revendications 1 à 4, caractérisé en ce que les feuilles comportent

EP 0 227 050 B1

pour la formulation de la forme creuse une zone non scellée dans laquelle une feuille dépasse de l'autre de quelques millimètres.

8. Procédé selon la revendication 6, caractérisé en ce que la masse de formulation et l'agent de séparation présentent des affinités opposées.

9. Procédé selon la revendication 6, caractérisé en ce que dans le cas d'une masse de formulation lipophile ou hydrophobe, avant de l'introduire, on envoie de l'eau nébulisée dans la forme creuse ou dans l'ampoule.

10. Procédé selon la revendication 6, caractérisé en ce qu'en cas de masses de formulation hydrophiles, on recouvre la surface intérieure de la forme creuse ou de l'ampoule d'huiles hydrophobes.

11. Procédé selon l'une des revendications 1 à 4 et 6 à 10, caractérisé en ce que la forme creuse ou l'ampoule est en matière thermoplastique, aluminium enduit de matière thermoplastique ou en papier doublé de matière plastique.

12. Procédé selon l'une des revendications 1 à 4 et 6 à 11, caractérisé en ce qu'on applique l'agent de séparation, déjà avant la formation de la forme creuse ou de l'ampoule, d'un côté de la feuille à mettre en forme, cette face constituant ultérieurement la face intérieure de la forme creuse ou de l'ampoule.

13. Procédé selon l'une des revendications 1 à 4 et 6 à 12, caractérisé en ce qu'on applique à la surface intérieure de la forme creuse ou de l'ampoule des agents ayant un effet favorable sur l'application et/ou l'acceptance de la masse de formulation finie.

14. Procédé selon la revendication 13, caractérisé en ce que les agents constituent des colorants, des arômes, des édulcorants des agents de conservation et/ou des lubrifiants.

15. Procédé selon l'une des revendications 1 à 14, caractérisé en ce qu'on applique à un substrat une masse de formulation présentant un effet de retardement réglable, une masse de formulation présentant une résistance au suc gastrique ou une masse de formulation servant à l'application buccale ou sublinguale ou on l'indroduit dans la forme creuse ou l'ampoule.

16. Procédé selon l'une des revendications 1 à 15, caractérisé en ce qu' on produit un corps à mastiquer ou à sucer à partir de la masse de formulation.

17. Procédé selon l'une des revendications 1 à 4 et 6 à 16, caractérisé en ce qu'on remplit les formes creuses ou les ampoules sussessivement de masses de formulation différentes.

18. Procédé selon la revendication 17, caractérisé en ce que l'une des couches renferme la dose initiale et l'autre couche constitue de la formulation retardatrice.

19. Formes propres à l'administration de doses éventuelles comportant une masse de formulation thermoplastique d'un point de ramollissement supérieur à la température du corps, pouvant être obtenue par le procédé selon l'une au moins des revendications 1 à 18.

15

FIG. 1

FIG.2

a)

FIG.3

a)

EP 0 227 050 B1

18

# FIG.4